# EUROPÄISCHE PATENTSCHRIFT

(11) **EP 1 698 344 B1**
(45) Veröffentlichungstag und Bekanntmachung des Hinweises auf die Patenterteilung: **14.11.2007**
(21) Anmeldenummer: 06011271.1
(22) Anmeldetag: 24.02.2005
(51) Int. Cl.: A61K 36/00, A23L 1/30, A61K 33/06, A61K 33/34, A61K 31/59

(54) **Nahrungsergänzungsmittelpräparat**
Dietary supplement with piperin
Complément alimentaire comprenant de la pipérine

(43) Veröffentlichungstag der Anmeldung: 06.09.2006
(62) Teilanmeldung aus: 05004035.1
(73) Patentinhaber: PM-International AG, 1618 Luxembourg (LU)
(72) Erfinder: Schmitt, Gerhard, Dr., 64625 Bensheim (DE)
(74) Vertreter: Zech, Stefan Markus

(56) Entgegenhaltungen:
- US-A- 5 514 382
- US-A- 5 536 506
- US-A- 6 048 846
- US-A1- 2004 052 873
- US-A1- 2004 191 388
- US-B1- 6 440 450
- US-B1- 6 572 899

## Beschreibung

Die Erfindung betrifft ein Nahrungsergänzungsmittelpräparat enthaltend die Mineralstoffe und Spurenelemente (Wirkstoffe) Vitamin D, Calcium, Magnesium, Selen, Kupfer, Zink und Chrom.

Magnesium beeinflusst die Reizleitungsvorgänge im zentralen und peripheren Nervensystem.

Selen ist eine antioxidative Schutzsubstanz. Es ist Baustein in vielen Enzymsystemen.

Chrom unterstützt den Glucose-Toleranzfaktor und erhöht damit das Ansprechverhalten auf Insulin. Es erfüllt somit eine wichtige Funktion im Zuckerstoffwechsel.

Eisen ist ein Additiv, das sich unter anderem bei starker Übersäuerung als vorteilhaft erwiesen hat. Besonders Hochleistungssportler und Frauen sollten daher auf ein Nahrungsergänzungsmittelpräparat mit Eisenanteil zurückgreifen, um die Hämoglobinsynthese sicher zu stellen.

In US-A-5 536 506 werden eine Mischung und ein Verfahren zu ihrer Erstellung zur Verbesserung der Magen-Darm-Aufnahme und der systematischen Verwendung von Nahrungsstoffen bzw. Nahrungsergänzungsstoffen offenbart. Die Mischung weist dabei neben Piperin eine Vielzahl von unterschiedlichen Mineralstoffen und Spurenelementen auf.

US-A-6 048 846 beschreibt eine Zusammensetzung zur Behandlung des menschlichen Körpers, die eine Kombination von mindestens einem Hormon, mindestens einer Aminosäure, mindestens einem Enzym und/oder Vitamin und mindestens einem Mineralstoff umfasst. Dabei sind die Relationsverhältnisse des Hormons, der Aminosäure, des Enzyms und/oder Vitamins und des Mineralstoffs so aufeinander abgestimmt, dass diese Stoffe in optimalen Mengen im Körper gespeichert werden können.

In US 2004/0191388 A1 wird eine Zusammensetzung dargelegt, die als ein hydratisierendes Getränk verwendbar ist, die mindestens ein komplexes Kohlenhydrat, mindestens ein Chelat-Elektrolyt, Betain und Piperin, aufweist.

US-B1-6 572 899 erläutert eine Zusammensetzung, die eine wirkungsvolle Kombination von Nährstoffen enthält, die im Hinblick auf die Bekämpfung von Gedächtnisverlust, Demenzen und der Alzheimer-Krankheit zusammengestellt worden sind.

US-A-5 514 382 offenbart eine tägliche Vitamin- und Mineralstoffergänzung für Frauen, die unterschiedliche Vitamine, Spurenelemente und Mineralstoffe umfasst, wobei eine erste Variante dieser Ergänzung speziell für Frauen bis zum vierzigsten Lebensjahr und eine zweite Variante dieser Ergänzung speziell für Frauen ab dem vierzigsten Lebensjahr zusammengestellt worden ist.

Nachteilig bei bekannten Nahrungsergänzungsmittelpräparaten ist die hohe Zeitdauer bis zur Resorption (Aufnahme) der Mineralstoffe und Spurenelemente nach der Einnahme. Ferner können sich aufgrund ungenügender Abstimmung die enthaltenen Mineralstoffe und Spurenelemente gegenseitig behindern. Beispielsweise können die Resorptionskanäle für einen Mineralstoff bzw. ein Spurenelement durch ein oder mehrere andere Mineralstoffe bzw. Spurenelemente besetzt sein. Auch kann der Transport der einzelnen Wirkstoffe an die Wirkstätte durch andere Wirkstoffe behindert werden.

Hinzu kommt, dass mehrere Wirkstoffe untereinander nicht kompatibel sein können. Beispielsweise behindern sich Calcium und Magnesium gegenseitig, wenn ihr Mengenverhältnis und/oder ihre molekulare Einbindung nicht stimmen. Falsche Mengenverhältnisse und/oder molekulare Einbindung können beispielsweise zur Komplexbildung führen.

Aufgabe der vorliegenden Erfindung ist somit, ein Nahrungsergänzungsmittelpräparat mit verbesserten Resorptionseigenschaften für Mineralstoffe und Spurenelemente anzugeben. Ferner sollen die verschiedenen Mineralstoffe und Spurenelemente derart aufeinander abgestimmt sein, dass sie sich nicht gegenseitig in ihrer Resorption und in ihrem Transport an die Wirkungsstätte stören.

Diese Aufgabe wird gelöst durch ein Nahrungsergänzungsmittelpräparat mit den Merkmalen nach Anspruch 1. Vorteilhafte Weiterbildungen ergeben sich aus den abhängigen Ansprüchen.

Gemäß Anspruch 1 enthält das Nahrungsergänzungsmittelpräparat neben den Mineralstoffen und Spurenelementen Vitamin D, Calcium, Magnesium, Selen, Kupfer, Zink und Chrom mindestens einen Katalysator und/oder Wirkstoff (auch als REC - Resorption Enhancing Catalyst bezeichnet) und Magnesiumhydroxidcarbonat, wobei der Katalysator und/oder Wirkstoff die Resorption der Mineralstoffe und/oder Spurenelemente steigert und/oder verbessert und/oder beschleunigt.

Die Vorteile der Erfindung liegen insbesondere in den verbesserten Resorptionseigenschaften (einschließlich des verbesserten Transports der Wirkstoffe an ihre Wirkstätte) des Nahrungsergänzungsmittelpräparats. Dadurch werden die Mineralstoffe und/oder Spurenelemente schneller in den Körper aufgenommen und können mit geringerem Zeitabstand zur Einnahme ihre Wirkungen entfalten. Auch kann der Katalysator und/oder Wirkstoff die Wirksamkeit der Mineralstoffe und/oder Spurenelemente im Körper verbessern. Das Nahrungsergänzungsmittelpräparat eignet sich insbesondere dazu, Osteoporose und Übersäuerung entgegenzuwirken.

Viele der im Markt angebotenen Magnesiumverbindungen sind im Allgemeinen schwer resorbierbar. Üblich ist bisher vor allem die Verwendung von Magnesiumcarbonat. Demgegenüber führt die Verwendung von Magnesiumhydroxidcarbonat zu einer verbesserten Resorbierbarkeit des Magnesiums sowie zu einer Steigerung von dessen Wirksamkeit.

Es ist bevorzugt, dass der Katalysator und/oder Wirkstoff in Pulverform vorliegen.

Nach einer vorteilhaften Weiterbildung umfasst der Katalysator und/oder Wirkstoff ein Schwarzpfefferextrakt bzw. er besteht daraus. Aus Schwarzem Pfeffer (piper nigrum) als Ausgangssubstanz lässt sich ein besonders effektiver Katalysator und/oder Wirkstoff gewinnen.

Gemäß einer bevorzugten Ausführungsform umfasst der Katalysator und/oder Wirkstoff Piperin (eine chemische Variante ist 1-Piperoylpiperidin C₁₇H₁₉NO₃), besonders wirkungsvoll als Alkaloid, bzw. er besteht daraus. Aus Schwarzem Pfeffer (piper nigrum) als Ausgangssubstanz lässt sich ein Piperin gewinnen, das als Katalysator und/oder Wirkstoff besonders effektiv ist.

Von Vorteil ist, wenn der Piperinanteil im Katalysator und/oder Wirkstoff im Bereich von 90 Prozent bis 100 Prozent liegt, insbesondere im Bereich von 92 bis 100 Prozent, vorzugsweise bei mindestens 95 Prozent.

Eine Weiterbildung sieht vor, dass der Katalysator und/oder Wirkstoff vollständig oder zumindest weitestgehend in seiner molekularen Struktur, wie sie im Schwarzen Pfeffer vorliegt, belassen bleibt. Es soll sich dabei insbesondere um ein aus der Pflanze Schwarzer Pfeffer gewonnene Substanz und nicht um ein künstlich chemisch erzeugtes Produkt handeln. Eine spezielle Vermahlung kann die (zumindest weitestgehende) Aufrechterhaltung der natürlichen molekularen Struktur gewährleisten.

Zweckmäßigerweise enthält das Nahrungsmittelergänzungspräparat je nach Ausführungsvariante Beta-Carotin (Provitamin A) und/oder Kalium und/oder Natrium und/oder Eisen.

Eine bevorzugte Weiterbildung des Nahrungsmittelergänzungspräparat sieht vor, dass
der Calciumanteil im Präparat zwischen 1,6 und 17 Gew.% liegt, vorzugsweise zwischen 2,5 und 10 Gew.%, insbesondere bei 4,5 Gew.% und/oder
der Magnesiumanteil im Präparat zwischen 0,8 und 5,0 Gew.% liegt, vorzugsweise zwischen 2 und 3 Gew.%, insbesondere bei 2,2 Gew.%, und/oder
der Zinkanteil im Präparat zwischen 0,015 und 0,2 Gew.% liegt, vorzugsweise zwischen 0,05 und 0,1 Gew.%, insbesondere bei 0,08 Gew.%, und/oder
der Kupferanteil im Präparat zwischen 0,0015 und 0,017 Gew.% liegt, vorzugsweise zwischen 0,005 und 0,015 Gew.%, insbesondere bei 0,011 Gew.%, und/oder
der Chromanteil im Präparat zwischen 0,00015 und 0,0017 Gew.% liegt, vorzugsweise zwischen 0,0002 und 0,0008 Gew.%, insbesondere bei 0,00028 Gew.%, und/oder der Selenanteil im Präparat zwischen 0,00015 und 0,0012 Gew.% liegt, vorzugsweise zwischen 0,0002 und 0,0006 Gew.%, insbesondere bei 0,00028 Gew.%, und/oder der Vitamin-D-Anteil im Präparat zwischen 0,00001 und 0,00008 Gew.%, liegt, vorzugsweise zwischen 0,00002 und 0,00006 Gew.%, insbesondere bei 0,00004 Gew.%, und/oder
der Anteil des Katalysators und/oder Wirkstoffs zur Resorptionsverbesserung, insbesondere der Piperinanteil, im Präparat zwischen 0,15 und 1,7 Gew.% liegt, vorzugsweise zwischen 0,4 und 1,2 Gew.%, insbesondere bei 0,8 Gew.%.

Eine weitere bevorzugte Weiterbildung des Nahrungsmittelergänzungspräparat sieht vor, dass
der Kaliumanteil im Präparat zwischen 0,15 und 35 Gew.% liegt, vorzugsweise zwischen 0,5 und 10 Gew.%, insbesondere bei 0,8 Gew.%, und/oder
der Eisenanteil im Präparat bei nahezu 0 Gew.% oder zwischen 0,015 und 0,09 Gew.% liegt, vorzugsweise zwischen 0,03 und 0,09 Gew.%, insbesondere bei 0,08 Gew.%.

Zweckmäßige und bevorzugte Ausführungsvarianten sehen vor, dass das Nahrungsmittelergänzungspräparat in Pulverform vorliegt und/oder ausgebildet und bestimmt ist zur Herstellung einer wässrigen Lösung und/oder ausgebildet und bestimmt ist zur Herstellung eines Getränks, insbesondere eines Mineral-Basen-Gertränks. Als Getränk lässt sich das Präparat besonders einfach, flexibel und schnell einnehmen, wodurch eine schnelle Wirkung sichergestellt wird. Auch ermöglicht ein Getränk die problemlose und bequeme regelmäßige Verwendung, ohne die sich die gewünschten Wirkungen beim Menschen in der Regel nicht erzielen lassen.

Nachfolgend werden die charakteristischen Werte eines Ausführungsbeispiels des Nahrungsmittelergänzungspräparats gemäß der Erfindung angeführt.

Ausgehend von einer Nahrungsmittelergänzungspräparat-Portion von 6 g (Gramm), was der einzunehmenden Tagesmenge entspricht, gibt folgende Tabelle typische Mengenwerte der einzelnen Mineralstoffe bzw. Spurenelemente innerhalb dieser Portion an. Das heißt die Tagesportion von 6 g enthält 50 mg (Milligramm) Kalium, 270 mg Calcium, usw. Weitere verwendete Einheit: µg (Mikrogramm). Ferner sind die Grenzen angegeben, innerhalb derer die jeweilige Substanz variiert werden kann. Neben den angegebenen Substanzen sind in der Portion noch weitere Substanzen enthalten, die beispielsweise der Aromatisierung und/oder der Löslichkeit des Nahrungsmittelergänzungspräparats dienen. Dadurch wird eine guten Löslichkeit und eine geschmackliche Akzeptanz des Nahrungsmittelergänzungspräparats erreicht.

| Mineralstoff bzw. Spurenelement | Menge in einer 6-Gramm-Portion | Grenzwerte |
|---|---|---|
| Kalium | 50 mg | 10 mg - 2.000 mg |
| Calcium | 270 mg | 100 mg - 1.000 mg |
| Magnesium | 134 mg | 50 mg - 300 mg |
| Zink | 5 mg | 1 mg - 11 mg |
| Kupfer | 670 µg | 100 µg - 1 mg |
| Chrom | 17 µg | 10 µg - 100 µg |
| Selen | 17 µg | 10 µg - 70 µg |
| Eisen | 5 mg | 1 mg - 5 mg |
| Vitamin D | 2,5 µg | 1 µg - 5 µg |

Ferner kann die Menge an Piperin pro 6-Gramm-Portion typischerweise bei 0,1 mg bis 10 mg, vorzugsweise bei 0,3 mg bis 2 mg, insbesondere bei 0,5 mg liegen.

## Patentansprüche

1. Nahrungsergänzungsmittelpräparat enthaltend die Mineralstoffe und Spurenelemente Vitamin D, Calcium, Magnesium, Selen, Kupfer, Zink und Chrom,
**dadurch gekennzeichnet, dass**
das Präparat mindestens einen Katalysator und/oder Wirkstoff und Magnesiumhydroxidcarbonat enthält, wobei der Katalysator und/oder Wirkstoff die Resorption der Mineralstoffe und/oder Spurenelemente steigert und/oder verbessert und/oder beschleunigt.

2. Nahrungsmittelergänzungspräparat nach Anspruch 1,
**dadurch gekennzeichnet, dass**
der Katalysator und/oder Wirkstoff in Pulverform vorliegt.

3. Nahrungsmittelergänzungspräparat nach Anspruch 1 oder 2,
**dadurch gekennzeichnet, dass**
der Katalysator und/oder Wirkstoff ein Schwarzpfefferextrakt umfasst und/oder daraus besteht.

4. Nahrungsmittelergänzungspräparat nach einem der vorgehenden Ansprüche,
**dadurch gekennzeichnet, dass**
der Katalysator und/oder Wirkstoff Piperin (chemische Bezeichnung 1-Piperoylpiperidin C₁₇H₁₉NO₃), insbesondere als Alkaloid, umfasst und/oder daraus besteht, wobei das Piperin vorzugsweise aus Schwarzem Pfeffer gewonnen ist.

5. Nahrungsmittelergänzungspräparat nach Anspruch 4,
**dadurch gekennzeichnet, dass**
der Piperinanteil im Katalysator und/oder Wirkstoff im Bereich von 90 Gew.% bis 100 Gew.% liegt, insbesondere im Bereich von 92 bis 100 Gew.%, vorzugsweise bei mindestens 95 Gew.%.

6. Nahrungsmittelergänzungspräparat nach einem der vorhergehenden Ansprüche,
**dadurch gekennzeichnet, dass**
der Katalysator und/oder Wirkstoff vollständig oder zumindest weitestgehend in seiner molekularen Struktur, wie sie im Schwarzen Pfeffer vorliegt, belassen bleibt.

7. Nahrungsmittelergänzungspräparat nach einem der vorhergehenden Ansprüche,
**dadurch gekennzeichnet, dass**
der Calciumanteil im Präparat zwischen 1,6 und 17 Gew.% liegt, vorzugsweise zwischen 2,5 und 10 Gew.%, insbesondere bei 4,5 Gew.% und/oder
der Magnesiumanteil im Präparat zwischen 0,8 und 5,0 Gew.% liegt, vorzugsweise zwischen 2 und 3 Gew.%, insbesondere bei 2,2 Gew.%, und/oder
der Zinkanteil im Präparat zwischen 0,015 und 0,2 Gew.% liegt, vorzugsweise zwischen 0,05 und 0,1 Gew.%, insbesondere bei 0,08 Gew.%, und/oder
der Kupferanteil im Präparat zwischen 0,0015 und 0,017 Gew.% liegt, vorzugsweise zwischen 0,005 und 0,015 Gew.%, insbesondere bei 0,011 Gew.%,
und/oder
der Chromanteil im Präparat zwischen 0,00015 und 0,0017 Gew.% liegt, vorzugsweise zwischen 0,0002 und 0,0008 Gew.%, insbesondere bei 0,00028 Gew.%,
und/oder
der Selenanteil im Präparat zwischen 0,00015 und 0,0012 Gew.% liegt, vorzugsweise zwischen 0,0002 und 0,0006 Gew.%, insbesondere bei 0,00028 Gew.%,
und/oder
der Vitamin-D-Anteil im Präparat zwischen 0,00001 und 0,00008 Gew.%, liegt, vorzugsweise zwischen 0,00002 und 0,00006 Gew.%, insbesondere bei 0,00004 Gew.%, und/oder
der Anteil des Katalysators und/oder Wirkstoffs zur Resorptionsverbesserung, insbesondere der Piperinanteil, im Präparat zwischen 0,0015 und 0,17 Gew.% liegt, vorzugsweise zwischen 0,005 und 0,034 Gew.%, insbesondere bei 0,008 Gew.%.

8. Nahrungsmittelergänzungspräparat nach einem der vorhergehenden Ansprüche,
**dadurch gekennzeichnet, dass**
das Präparat Beta-Carotin (Provitamin A) und/oder Kalium und/oder Natrium und/oder Eisen enthält.

9. Nahrungsmittelergänzungspräparat nach Anspruch 8,
**dadurch gekennzeichnet, dass**
der Kaliumanteil im Präparat zwischen 0,15 und 35 Gew.% liegt, vorzugsweise zwischen 0,5 und 10 Gew.%, insbesondere bei 0,8 Gew.%, und/oder
der Eisenanteil im Präparat bei 0 Gew.% oder zwischen 0,015 und 0,09 Gew.% liegt, vorzugsweise zwischen 0,03 und 0,09 Gew.%, insbesondere bei 0,08 Gew.%.

10. Nahrungsmittelergänzungspräparat nach einem der vorhergehenden Ansprüche,
**dadurch gekennzeichnet, dass**
das Präparat in Pulverform vorliegt und/oder ausgebildet und bestimmt ist zur Herstellung einer wässrigen Lösung und/oder ausgebildet und bestimmt ist zur Herstellung eines Getränks, insbesondere eines Mineral-Basen-Getränks.

## Claims

1. A nutritional supplement containing the minerals and trace elements of vitamin D, calcium, magnesium, selenium, copper, zinc and chromium,
**characterized in that**
the supplement contains at least one catalyst and/or active substance and magnesium hydroxide carbonate, wherein the catalyst and/or active substance increases and/or improves and/or accelerates resorption of the minerals and/or trace elements.

2. The nutritional supplement according to claim 1,
**characterized in that**
the catalyst and/or active substance is in powdered form.

3. The nutritional supplement according to claim 1 or 2,
**characterized in that**
the catalyst and/or active substance comprises and/or consists of a black pepper extract.

4. The nutritional supplement according to any one of the preceding claims,
**characterized in that**
the catalyst and/or active substance comprises and/or consists of piperine (chemical name 1-piperoylpiperidine C₁₇H₁₉NO₃), in particular as an alkaloid, wherein said piperine is preferably extracted from black pepper.

5. The nutritional supplement according to claim 4,
**characterized in that**
the piperine content in the catalyst and/or active substance is in the range of between 90% and 100% by weight, in particular in the range of between 92% and 100% by weight, preferably at least 95% by weight.

6. The nutritional supplement according to any one of the preceding claims,
**characterized in that** the catalyst and/or active substance completely or at least to the greatest extent possible retains its molecular structure as present in black pepper.

7. The nutritional supplement according to any one of the preceding claims,
**characterized in that**
the supplement has a calcium content of between 1.6% and 17% by weight, preferably between 2.5% and 10% by weight, in particular of 4.5% by weight, and/or
the supplement has a magnesium content of between 0.8% and 5% by weight, preferably between 2% and 3% by weight, in particular of 2.2% by weight, and/or
the supplement has a zinc content of between 0.015% and 0.2% by weight, preferably between 0.05% and 0.1% by weight, in particular of 0.08% by weight, and/or
the supplement has a copper content of between 0.0015% and 0.017% by weight, preferably between 0.005% and 0.015% by weight, in particular of 0.011% by weight,
and/or
the supplement has a chromium content of between 0.00015% and 0.0017% by weight, preferably between 0.0002% and 0.0008% by weight, in particular of 0.00028% by weight,
and/or
the supplement has a selenium content of between 0.00015% and 0.0012% by weight, preferably between 0.0002% and 0.0006% by weight, in particular of 0.00028% by weight,
and/or
the supplement has a vitamin D content of between 0.00001% and 0.00008% by weight, preferably between 0.00002% and 0.00006% by weight, in particular of 0.00004 % by weight, and/or the content of the catalyst and/or active substance for enhanced resorption in the supplement, in particular the piperine content, is between 0.0015% and 0.17% by weight, preferably between 0.005% and 0.034% by weight, in particular 0.008% by weight.

8. The nutritional supplement according to any one of the preceding claims,
**characterized in that**
the supplement contains beta-carotene (provitamin A) and/or potassium and/or sodium and/or iron.

9. The nutritional supplement according to claim 8,
**characterized in that** the supplement has a potassium content of between 0.15% and 35% by weight, preferably between 0.5% and 10% by weight, in particular of 0.8% by weight, and/or the supplement has an iron content of 0% by weight or between 0.015% and 0.09% by weight, preferably between 0.03% and 0.09% by weight, in particular of 0.08% by weight.

10. The nutritional supplement according to any one of the preceding claims,
**characterized in that**
the supplement is in powdered form and/or is developed and used to produce an aqueous solution and/or is developed and used to produce a beverage, in particular a mineral-based beverage.

## Revendications

1. Préparation de complément alimentaire contenant les minéraux et les oligo-éléments vitamine D, calcium, magnésium, sélénium, cuivre, zinc et chrome,
**caractérisée en ce que**
la préparation contient au moins un catalyseur et/ou substance active et hydroxyde-carbonate de magnésium, le catalyseur et/ou la substance active augmentant et/ou améliorant et/ou accélérant la résorption des minéraux et/ou des oligo-éléments.

2. Préparation de complément alimentaire selon la revendication 1,
**caractérisée en ce que**
le catalyseur et/ou la substance active se présentent sous forme pulvérulente.

3. Préparation de complément alimentaire selon la revendication 1 ou 2,
**caractérisée en ce que**
le catalyseur et/ou la substance active comprennent/comprend un extrait de poivre noir et/ou en est constitué ou en sont constitués.

4. Préparation de complément alimentaire selon l'une quelconque des revendications précédentes,
**caractérisée en ce que**
le catalyseur et/ou la substance active comprend ou comprennent et/ou est constitué ou sont constitués de pipérine (désignation chimique 1-piperoylpiperidin C₁₇H₁₉NO₃), en particulier en tant qu'alcaloïde, la pipérine provenant de préférence de poivre noir.

5. Préparation de complément alimentaire selon la revendication 4,
**caractérisée en ce que**
la proportion de pipérine dans le catalyseur et/ou la substance active est comprise entre 90 % en poids et 100 % en poids, en particulier entre 92 % en poids et 100 % en poids, de préférence d'au moins 95 % en poids.

6. Préparation de complément alimentaire selon l'une quelconque des revendications précédentes,
**caractérisée en ce que**
le catalyseur et/ou la substance active demeure ou demeurent totalement ou, au moins, largement dans sa structure moléculaire telle que présente dans le poivre noir.

7. Préparation de complément alimentaire selon l'une quelconque des revendications précédentes,
**caractérisée en ce que**
la proportion de calcium dans la préparation est comprise entre 1,6 et 17 % en poids, de préférence entre 2,5 et 10 % en poids, en particulier de 4,5 % en poids et/ou
la proportion de magnésium dans la préparation est comprise entre 0,8 et 5 % en poids, de préférence entre 2 et 3 % en poids, en particulier de 2,2 % en poids et/ou
la proportion de zinc dans la préparation est comprise entre 0,015 et 0,2 % en poids, de préférence entre 0,05 et 0,1 % en poids, en particulier de 0,08 % en poids et/ou
la proportion de cuivre dans la préparation est comprise entre 0,0015 et 0,017 % en poids, de préférence entre 0,005 et 0,015 % en poids, en particulier de 0,011 % en poids et/ou
la proportion de chrome dans la préparation est comprise entre 0,00015 et 0,0017 % en poids, de préférence entre 0,0002 et 0,0008 % en poids, en particulier de 0,00028 % en poids et/ou
la proportion de sélénium dans la préparation est comprise entre 0,00015 et 0,0012 % en poids, de préférence entre 0,0002 et 0,0006 % en poids, en particulier de 0,00028 % en poids et/ou
la proportion de vitamine D dans la préparation est comprise entre 0,00001 et 0,00008 % en poids, de préférence entre 0,00002 et 0,00006 % en poids, en particulier de 0,00004 % en poids et/ou
la proportion de catalyseur et/ou de la substance active pour l'amélioration de la résorption, en particulier la proportion de pipérine, dans la préparation est comprise entre 0,0015 et 0,17 % en poids, de préférence entre 0,005 et 0,034 % en poids, en particulier de 0,008 % en poids.

8. Préparation de complément alimentaire selon l'une quelconque des revendications précédentes,
**caractérisée en ce que**
la préparation contient de la béta-carotine (provitamine A) et/ou du potassium et/ou du sodium et/ou du fer.

9. Préparation de complément alimentaire selon la revendication 8,
**caractérisée en ce que**
la proportion de potassium dans la préparation est comprise entre 0,15 et 35 % en poids, de préférence entre 0,5 et 10 % en poids, en particulier de 0,8 % en poids et/ou
la proportion de fer dans la préparation est de 0 % en poids ou comprise entre 0,015 et 0,09 % en poids, de préférence entre 0,03 et 0,09 % en poids, en particulier de 0,08 % en poids.

10. Préparation de complément alimentaire selon l'une quelconque des revendications précédentes,
**caractérisée en ce que**
la préparation est présente et/ou est constituée en forme pulvérulente et est déterminée pour la préparation d'une solution aqueuse et/ou est constituée et déterminée pour la préparation d'une boisson, en particulier d'une boisson à bases minérales.
